**Europäisches Patentamt**

**European Patent Office**

**Office européen des brevets**

(19)

(11) Veröffentlichungsnummer: **0 127 133**
**B1**

(12)

# EUROPÄISCHE PATENTSCHRIFT

(45) Veröffentlichungstag der Patentschrift:
16.03.88

(21) Anmeldenummer: 84105908.2

(22) Anmeldetag: 24.05.84

(51) Int. Cl.⁴: **C 07 D 277/82**

(54) Verfahren zur Herstellung von 2-Acetylamino-6-nitro-benzthiazol und 2-Amino-6-nitro-benzthiazol.

(30) Priorität: 28.05.83 DE 3319507

(43) Veröffentlichungstag der Anmeldung:
05.12.84 Patentblatt 84/49

(45) Bekanntmachung des Hinweises auf die Patenterteilung:
16.03.88 Patentblatt 88/11

(84) Benannte Vertragsstaaten:
BE CH DE FR GB IT LI

(56) Entgegenhaltungen:
EP-A-0 039 835
DE-A-2 601 700
US-A-2 833 689

**Die Akte enthält technische Angaben, die nach dem Eingang der Anmeldung eingereicht wurden und die nicht in dieser Patentschrift enthalten sind.**

(73) Patentinhaber: HOECHST AKTIENGESELLSCHAFT,
Postfach 80 03 20, D-6230 Frankfurt am Main 80
(DE)

(72) Erfinder: Tronich, Wolfgang, Dr., Adolf- Guckes-Weg 5, D-6239 Eppstein/Taunus (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Es ist aus der Deutschen Offenlegungsschrift 30 18 028 bekannt, 2-Amino-6-nitro-benzthiazol in der Weise herzustellen, daß man 2-Acetylamino-benzthiazol in einem Schwefelsäure/Salpetersäure-Gemisch oder in Salpetersäure selbst nitriert, das Nitrierungsprodukt anschließend isoliert und mittels Säure oder Alkalien, vorzugsweise in wößrig-alkoholischem Medium, zum 2-Amino-6-nitro-benzthiazol verseift. Entscheidende Nachteile dieses bekannten Verfahrens der Deutschen Offenlegungsschrift 30 18 028 bestehen darin, daß daß als Ausgangsverbindung dienende 2-Acetylamino-benzthiazol - gemäß den Angaben in der Beschreibung und dem Beispiel 1 - nach der Verfahrensweise aus Liebigs Annalen 212, 326 (1882) zunächst recht aufwendig hergestellt, gereinigt und getrocknet wird, wobei die Reinigung und insbesondere die unumgängliche Trocknung einen zusätzlichen Arbeitsaufwand und somit zusätzliche Kosten verursachen, und weiterhin, daß das als Zwischenstufe hergestellte 2-Acetylamino-6-nitro-benzthiazol vor dessen Hydrolyse zum 2-Amino-6-nitro-benzthiazol, die bevorzugt in alkalisch-methanolischem Medium durchgeführt wird, zwischenisoliert und mit viel Wasser säurefrei gewaschen werden muß und daß daraufhin eine erneute Isolierung, nunmehr des gebildeten 2-Amino-6-nitro-benzthiazols nach der Hydrolysereaktion, und Wäsche des Produktes mit Methanol und Wasser bis zur Alkalifreiheit erfolgen muß.

Mit der vorliegenden Erfindung wurde nun ein Verfahren gefunden, das die Nachteile des Verfahrens der Deutschen Offenlegungsschrift 30 18 028 beseitigt und mit welchem 2-Acetylamino-6-nitro-benzthiazol als Vorstufe von 2-Amino-6-nitro-benzthiazol und das 2-Amino-6-nitro-benzthiazol selbst in einer verfahrenstechnisch wesentlich günstigeren und einfacheren Weise hergestellt werden können.

Dieses erfindungsgemäße Verfahren zur Herstellung von 2-Acetylamino-6-nitro-benzthiazol und von 2-Amino-6-nitro-benzthiazol ist dadurch gekennzeichnet, daß man in, bevorzugt 98 - 100 gew.-%-iger, Schwefelsäure als Reaktionsmedium 2-Amino-benzthiazol zunächst mit der äquivalenten Menge an Essigsäureanhydrid oder mit einem geringen Überschuß an Essigsäureanhydrid, wie mit einer 1,05- bis 1,3-fach, vorzugsweise 1,05- bis 1,2-fach, äquivalenten Menge an Essigsäureanhydrid, umsetzt und sodann ohne Zwischenisolierung das 2-Acetylamino-benzthiazol mit wäßriger Salpetersäure in vorzugsweise äquivalenter Menge oder in einem geringen Überschuß, wie zum Beispiel mit einer bis zu 1,1-fach, gegebenenfalls bis zu 1,3-fach äquivalenten Menge an wäßriger Salpetersäure, bezogen auf das eingesetzte 2-Amino-benzthiazol, nitriert und gewünschtenfalls das 2-Acetylamino-6-nitro-benzthiazol ohne Zwischenisolierung nach Verdünnen mit Wasser zum 2-Amino-6-nitro-benzthiazol hydrolysiert.

Selbstverständlich kann das im erfindungsgemäßen Verfahren hergestellte 2-Acetylamino-6-nitro-benzthiazol aus dem Reaktionsansatz in üblicher Weise isoliert und sodann gemäß bekannter Verfahrensweise zum 2-Amino-6-nitro-benzthiazol hydrolysiert werden. Das hier bereit gestellte erfindungsgemäße Verfahren erlaubt es jedoch in ganz besonders vorteilhafter Weise, sofern gewünscht, das im Reaktionsansatz vorhandene 2-Acetylamino-6-nitro-benzthiazol nach der Nitrierungsreaktion nicht zu isolieren und es mit Hilfe der im Reaktionsansatz vorhandenen wäßrigen Schwefelsäure nach weiterem Verdünnen der Schwefelsäure mit Wasser (eine Vorverdünnung der Schwefelsäure erfolgte bereits durch Zugabe der wäßrigen Salpetersäure und durch Bildung des Reaktionswassers) im gleichen Reaktionsansatz ohne Zwischenisolierung bei verringerter Schwefelsäurekonzentration zu hydrolysieren. Das gebildete 2-Amino-6-nitro-benzthiazol kann sodann in üblicher Weise isoliert werden, bevorzugt durch weitere Verdünnung des Reaktionsansatzes mit Wasser, wobei die Wassermenge in weiten Grenzen variiert werden kann, und durch Auskristallisation unter Abkühlung mit anschließender Filtration.

Das erfindungsgemäße Verfahren sowohl zur Herstellung des 2-Acetylamino-6-nitro-benzthiazols als auch des 2-Amino-6-nitro-benzthiazols wird bevorzugt als Eintopf-Reaktion durchgeführt.

In der Regel verwendet man bei der Acetylierungsreaktion die als Reaktionsmedium dienende Schwefelsäure in einer 3- bis 7-fachen Gewichtsmenge, bezogen auf die 2-Amino-benzthiazol-Ausgangsverbindung. Die Acetylierung wird in einem Temperaturbereich zwischen 5 bis 40° C durchgeführt. Vorzugsweise führt man die Acetylierungsreaktion bei einer Temperatur zwischen 10 und 30° C aus.

Die als Nitrierungsmittel dienende Salpetersäure wird in der Regel als 50 - 90 gew.-%ige wäßrige Salpetersäure, vorzugsweise als 55 - 70 gew.-%-ige Salpetersäure, eingesetzt. Die Nitrierung wird bei einer Temperatur zwischcn 5 und 40° C ausgeführt vorzugsweise verwendet man eine Nitrierungstemperatur zwischen 10 und 30° C.

Die Hydrolyse des 2-Acetylamino-6-nitro-benzthiazols zum 2-Amino-6-nitro-benzthiazol in dem wäßrig-schwefelsauren Medium erfolgt bei einer Temperatur von oberhalb 40° C; vorzugsweise wird die Hydrolysereaktion bei einer Temperatur zwischen 65 und 90° C durchgeführt. Eine für die Hydrolyse günstige Schwefelsäurekonzentration ist eine solche, bei der die Schwefelsäure im Ansatz eine 50 - 60 gew.-%-ige wäßrige Schwefelsäure, bezogen auf den Gesamtgehalt Wasser/$H_2SO_4$ im Ansatz, vorliegt. Dementsprechend gibt man das Wasser in einer solchen Menge zu, daß die Schwefelsäurekonzentration in diesem Bereich, vorzugsweise bei 53 - 57 Gew.-%, liegt.

Das erfindungsgemäße Verfahren liefert in einfacher und vorteilhafter Weise ohne Zwischenisolierung der Zwischenprodukte, insbesondere auch in der Eintopf-Verfahrensweise, 2-Amino-6-nitro-benzthiazol in uter Ausbeute von größer als 75 % d.Th., bezogen auf die 2-Amino-benzthiazol-Ausgangsverbindung, und in guter Reinheit, die den Anforderungen der Weiterverarbeitung des 2-Amino-6-nitro-benzthiazols zu Farbstoffen, insbesondere Azopigmenten, genügt.

Die nachfolgenden Beispiele dienen zur Erläuterung der Erfindung. Die Teile sind Gewichtsteile und die Prozentangaben beziehen sich auf Gewichtsprozente, sofern nicht anders vermerkt.

**Beispiel 1**

150 Teile 2-Amino-benzthiazol werden bei einer Temperatur zwischen 20 und 25° C in 460 Teile 100 %-iger Schwefelsäure eingetragen. Diese Mischung wird bei einer Temperatur zwischen 20 und 25°C innerhalb von 20 Minuten mit 120 Teilen Essigsäureanhydrid versetzt. Man rührt diesen Ansatz etwa 6 Stunden bei einer Temperatur zwischen 20 und 25°C weiter und gibt danach in diesem Temperaturbereich innerhalb von 4 Stunden 105 Teile einer 60 %-igen wäßrigen Salpetersäure hinzu. Dieser Nitrierungsansatz wird sodann noch weitere 3 bis 4 Stunden bei 20 bis 25° C nachgerührt.

Zur Aufarbeitung des gebildeten 2-Acetylamino-6-nitro-benzthiazols kann das Reaktionsgemisch anschließend auf Eis gegossen werden. Das ausgefallene Produkt wird abfiltriert, mit Wasser gut gewaschen und getrocknet. Ausbeute: 192 Teile 2-Acetylamino-6-nitro-benzthiazol mit einem Schelzpunkt von 287 - 289°C (entsprechend 81 % d.Th.).

**Beispiel 2**

Zur erfindungsgemäßen Herstellung des 2-Amino-6-nitro-benzthiazols verfährt man gemäß der Verfahrensweise des Beispieles 1, jedoch ohne Isolierung dieses Nitrierungsproduktes, in dem man den Nitrierungsansatz des Beispieles 1 nach beendeter Nitrierung mit etwa 320 Teilen Wasser versetzt und die Hydrolyse unter Rühren bei einer Temperatur zwischen 70 und 80°C während etwa 2 Stunden durchführt.

Nach beendeter Hydrolyse wird der Ansatz mit 1200 Teilen Wasser vermischt. Man läßt die noch warme Lösung unter Rühren erkalten und isoliert das auskristallisierte 2-Amino-6-nitro-benzthiazol durch Filtration, wäscht es mit Wasser und trocknet es. Ausbeute: 152 Teile 2-Amino-6-nitro-benzthiazol mit einem Schmelzpunkt von 247 - 248°C (entsprechend 78 % d.Th.). Das so erhältliche 2-Amino-6-nitro-benzthiazol kann beispielsweise als Diazokomponente zur Herstellung von Azoverbindungen dienen. Es löst sich in wäßriger 80 %-iger Phosphorsäure mit klarer, hellbrauner Farbe; in dieser Lösung läßt es sich in bekannter Weise in die Diazoniumverbindung überführen, ohne daß eine zusätzliche Reinigung erforderlich ist.

Die Isolierung des 2-Amino-6-nitro-benzthiazols kann nach Verdünnen des Ansatzes mit den 1200 Teilen warmem Wasser auch in der Weise erfolgen, daß man diesen verdünnten Ansatz durch Zugabe von wäßriger Natronlauge auf einen pH-Wert zwischen 2 und 3 einstellt, das ausgefallene Produkt isoliert, mit Wasser wäscht und trocknet. Ausbeute: 162 Teile 2-Amino-6-nitro-benzthiazol mit einem Schmelzpunkt von 243° C. Es ist ebenfalls in etwa 80 %-iger Phosphorsäure klar löslich.

**Patentansprüche**

1.Verfahren zur Herstellung von 2-Acetylamino-6-nitro-benzthiazol und von 2-Amino-6-nitro-benzthiazol, dadurch gekennzeichnet, daß man in Schwefelsäure als Reaktionsmedium 2-Amino-benzthiazol mit der äquivalenten Menge an Essigsäureanhydrid oder mit einem geringen Überschuß an Essigsäureanhydrid bei einer Temperatur zwischen 5 und 40°C umsetzt und sodann ohne Zwischenisolierung das 2-Acetylamino-benzthiazol mit der äquivalenten Menge einer wäßrigen Salpetersäure oder mit einem geringen Überschuß an wäßriger Salpetersäure bei einer Temperatur zwischen 5 und 40°C nitriert und gewünschtenfalls das 2-Acetylamino-6-nitro-benzthiazol ohne Zwischenisolierung nach Zusatz von Wasser zum 2-amino-6-nitro-benzthiazol hydrolysiert.

2. Verfahren nach Anspruch 1, dadurch gekennzeichnet, daß Essigsäureanhydrid in der 1,0- bis 1,3-fach äquivalenten Menge verwendet wird.

3. Verfahren nach Anspruch 1 oder 2, dadurch gekennzeichnet, daß die Acetylierung bei einer Temperatur zwischen 10 und 30°C erfolgt.

4. Verfahren nach Anspruch 1, 2 oder 3, dadurch gekennzeichnet, daß 55 - 70 gew.%-ige Salpetersäure verwendet wird.

5. Verfahren nach Anspruch 4, dadurch gekennzeichnet, daß die Nitrierung bei einer Temperatur zwischen 10 und 30°C erfolgt.

6. Verfahren nach Anspruch 4 und 5, dadurch gekennzeichnet, daß die wäßrige Salpetersäure in der 1,0- bis 1,3-fach äquivalenten Menge, bezogen auf 2-Amino-benzthiazol, als Reaktionskomponente verwendet wird.

7. Verfahren nach mindestens eidem der Ansprüche 1 bis 6, dadurch gekennzeichnet, daß man bei der Herstellung des 2-Amino-6-nitro-benzthiazols die Hydrolyse ohne Zwischenisolierung des 2-Acetylamino-6-nitro-benzthiazols in wäßrig-schwefelsaurer Lösung durchführt.

8. Verfahren nach mindestens einem der Ansprüche 1 bis 7, dadurch gekennzeichnet, daß die Umsetzungen in einer Eintopf-Verfahrensweise durchgeführt werden.

## Claims

1. A process for the preparation of 2-acetylamino-6-nitrobenzothiazole and of 2-amino-6-nitrobenzothiazole, characterized by reacting 2-aminobenzothiazole in sulfuric acid as the reaction medium with the equivalent amount of acetic anhydride or with a slight excess of acetic anhydride at a temperature between 5 and 40°C, and then, without intermediate isolation of the 2-acetylaminobenzothiazole, nitrating it with the equivalent amount of aqueous nitric acid or with a slight excess of aqueous nitric acid at a temperature between 5 and 40°C, and, if desired, hydrolyzing the 2-acetylamino-6-nitrobenzothiazole, without intermediate isolation, after addition of water, to give 2-amino-6-nitrobenzothiazole.

2. The process as claimed in claim 1, wherein 1.0 to 1.3 times the equivalent amount of acetic anhydride is used.

3. The process as claimed in claim 1 or 2, wherein the acetylation is carried out at a temperature between 10 and 30°C.

4. The process as claimed in claim 1, 2 or 3, wherein 55 - 70 % by weight nitric acid is used.

5. The process as claimed in claim 4, wherein the nitration is carried out at a temperature between 10 and 30°C.

6. The process according to claims 4 and 5, wherein the aqueous nitric acid is used as reaction component in 1.0 to 1.3 times the equivalent amount related to 2-aminobenzothiazole.

7. The process as claimed in at least one of claims 1 to 6, wherein, for the preparation of 2-amino-6-nitrobenzothiazole, the hydrolysis is carried out in aqueous sulfuric acid solution without intermediate isolation of the 2-acetylamino-6-nitrobenzothiazole.

8. The process as claimed in at least one of claims 1 to 7, wherein the reactions are carried out in a one-pot procedure.

## Revendications

1. Procédé pour préparer l'acétylamino-2 nitro-6 benzothiazole et l'amino-2 nitro-6 benzothiazole, procédé caractérisé en ce qu'on fait réagir l'amino-2 benzothiazole, dans un milieu réactionnel constitué d'acide sulfurique, avec la quantité équivalente d'anhydride acétique ou avec un léger excès d'anhydride acétique, à une température comprise entre 5 et 40°C, et, sans l'isoler intermédiairement, on nitre l'acétylamino-2 benzothiazole avec la quantité équivalente d'un acide nitrique aqueux ou avec un léger excès d'un acide nitrique aqueux, à une température comprise entre 5 et 40°C, et, si on le désire, sans l'isoler intermédiairement on hydrolyse l'acétylamino-2 nitro-6 benzothiazole, après addition d'eau, et on le convertit ainsi en l'amino-2 nitro-6 benzothiazole.

2. Procédé selon la revendication 1 caractérisé en ce que l' anhydride acétique est utilisé en une quantité représentant de 1,0 à 1,3 fois la quantité équivalente.

3. Procédé selon l'une des revendications 1 et 2, caractérisé en ce que l'acétylation est effectuée à une température comprise entre 10 et 30°C.

4. Procédé selon l'une quelconque des revendications 1, 2 et 3, caractérisé en ce qu'on utilise un acide nitrique d'une concentration comprise entre 55 et 70 % en poids.

5. Procédé selon la revendication 4 caractérisé en ce que la nitration est effectuée à une température comprise entre 10 et 30°C.

6. Procédé selon l'une des revendications 4 et 5, caractérisé en ce que l'acide nitrique aqueux est utilisé, comme composante réactionnelle, en une quantité représentant de 1,0 à 1,3 fois la quantité équivalente, par rapport à l'amino-2 benzothiazole.

7. Procédé selon une ou plusieurs des revendications 1 à 6, caractérisé en ce que, dans la préparation de l'amino-2 nitro-6 benzothiazole, on effectue l'hydrolyse, sans isoler intermédiairement l'acétylamino-2 nitro-6 benzothiazole, sur une solution aqueuse rendue acide par de l'acide sulfurique.

8. Procédé selon une ou plusieurs des revendications 1 à 7, caractérisé en ce que les réactions sont effectuées suivant le mode "en un seul pot".